# EUROPEAN PATENT SPECIFICATION

(11) **EP 1 950 296 B1**
(45) Date of publication and mention of the grant of the patent: **07.05.2014**
(21) Application number: 07021557.9
(22) Date of filing: 12.01.2001
(51) Int. Cl.: C12N 15/11, A61K 31/7088, A61P 37/04

(54) **Oligodeoxynucleotide and its use to induce an immune response**
Oligodeoxynukleotid und seine Verwendung zur Induktion einer Immunreaktion
Oligodéoxynucléotide et son utilisation pour induire une réponse immunitaire

(30) Priority: 14.01.2000 US 176115 P
(43) Date of publication of application: 30.07.2008
(62) Divisional of application: 01902045.2
(73) Proprietor: The Government of the United States of America, as represented by the Secretary of Health and Human Services, Rockville, MD 20852-3804 (US)
(72) Inventor: Klinman, Dennis, Potomac, MD 20854 (US); Ishi, Ken, Columbia, MD 21045 (US); Verthelyi, Daniela, Potomac, MD 20854 (US)
(74) Representative: Walker, Ross Thomson

(56) References cited:
- WO-A1-98/40100
- JAHN-SCHMID B ET AL: "Oligodeoxynucleotides containing CpG motifs modulate the allergic T H2 response of BALB/c mice to Bet v 1, the major birch pollen allergen", JOURNAL OF ALLERGY AND CLINICAL IMMUNOLOGY, MOSBY, INC, US, vol. 104, no. 5, 1 November 1999 (1999-11-01), pages 1015-1023, XP027421057, ISSN: 0091-6749, DOI: DOI:10.1016/S0091-6749(99)70083-7 [retrieved on 1999-11-01]

### SEQUENCE LISTING

<110> THE GOVERNMENT OF THE UNITED STATES OF AMERICA,
   REPRESENTED BY THE SECRETARY, DEPARTMENT
   OF HEALTH AND HUMAN SERVICES
   KLINMAN, Dennis
   ISHII, Ken
   VERTHELYI, Daniela
<120> OLIGODEOXYNUCLEOTIDE AND ITS USE TO INDUCE AN IMMUNE RESPONSE
<130> 207509
<140> WO
   <141> 2001-01-12
<150> US 60/176,115
   <151> 2000-01-14
<160> 112
<170> PatentIn Ver. 2.1
<210> 1
   <211> 12
   <212> DNA
   <213> Artifical Sequence
<220>
   <223> Description of Artificial Sequence: Synthetic DNA
<400> 1
   tcgagcgttc tc 12
<210> 2
   <211> 19
   <212> DNA
   <213> Artifical Sequence
<220>
   <223> Description of Artificial Sequence: Synthetic DNA
<400> 2
   atcgactctc gagcgttct 19
<210> 3
   <211> 24
   <212> DNA
   <213> Artifical Sequence
<220>
   <223> Description of Artificial Sequence: Synthetic DNA
<400> 3
   tcgtcgtttt gtcgttttgc tgtt 24
<210> 4
   <211> 14
   <212> DNA
   <213> Artifical Sequence
<220>
   <223> Description of Artificial Sequence: Synthetic DNA
<400> 4
   tctcgagcgt tctc 14
<210> 5
   <211> 19
   <212> DNA
   <213> Artifical Sequence
<220>
   <223> Description of Artificial Sequence: Synthetic DNA
<400> 5
   tcgactctcg agcgttctc 19
<210> 6
   <211> 20
   <212> DNA
   <213> Artifical Sequence
<220>
   <223> Description of Artificial Sequence: Synthetic DNA
<400> 6
   atcgactagc gttcgttctc 20
<210> 7
   <211> 16
   <212> DNA
   <213> Artifical Sequence
<220>
   <223> Description of Artificial Sequence: Synthetic DNA
<400> 7
   actctcgagc gttctc 16
<210> 8
   <211> 15
   <212> DNA
   <213> Artifical Sequence
<220>
   <223> Description of Artificial Sequence: Synthetic DNA
<400> 8
   ctctcgagcg ttctc 15
<210> 9
   <211> 12
   <212> DNA
   <213> Artifical Sequence
<220>
   <223> Description of Artificial Sequence: Synthetic DNA
<400> 9
   gtcgacgttg ac 12
<210> 10
   <211> 12
   <212> DNA
   <213> Artifical Sequence
<220>
   <223> Description of Artificial Sequence: Synthetic DNA
<400> 10
   gtcggcgttg ac 12
<210> 11
   <211> 18
   <212> DNA
   <213> Artifical Sequence
<220>
   <223> Description of Artificial Sequence: Synthetic DNA
<400> 11
   cgactctcga gcgttctc 18
<210> 12
   <211> 12
   <212> DNA
   <213> Artifical Sequence
<220>
   <223> Description of Artificial Sequence: Synthetic DNA
<400> 12
   gtcgacgctg ac 12
<210> 13
   <211> 12
   <212> DNA
   <213> Artifical Sequence
<220>
   <223> Description of Artificial Sequence: Synthetic DNA
<400> 13
   gtcagcgttg ac 12
<210> 14
   <211> 17
   <212> DNA
   <213> Artifical Sequence
<220>
   <223> Description of Artificial Sequence: Synthetic DNA
<400> 14
   gactctcgag cgttctc 17
<210> 15
   <211> 12
   <212> DNA
   <213> Artifical Sequence
<220>
   <223> Description of Artificial Sequence: Synthetic DNA
<400> 15
   gtcgtcgatg ac 12
<210> 16
   <211> 20
   <212> DNA
   <213> Artifical Sequence
<220>
   <223> Description of Artificial Sequence: Synthetic DNA
<400> 16
   atgcactctc gagcgttctc 20
<210> 17
   <211> 13
   <212> DNA
   <213> Artifical Sequence
<220>
   <223> Description of Artificial Sequence: Synthetic DNA
<400> 17
   ctcgagcgtt ctc 13
<210> 18
   <211> 12
   <212> DNA
   <213> Artifical Sequence
<220>
   <223> Description of Artificial Sequence: Synthetic DNA
<400> 18
   tgcagcgttc tc 12
<210> 19
   <211> 12
   <212> DNA
   <213> Artifical Sequence
<220>
   <223> Description of Artificial Sequence: Synthetic DNA
<400> 19
   tttggcgttt tt 12
<210> 20
   <211> 20
   <212> DNA
   <213> Artifical Sequence
<220>
   <223> Description of Artificial Sequence: Synthetic DNA
<400> 20
   atcgactctc gagcgttctc 20
<210> 21
   <211> 20
   <212> DNA
   <213> Artifical Sequence
<220>
   <223> Description of Artificial Sequence: Synthetic DNA
<400> 21
   agcgtttctc gatcgacctc 20
<210> 22
   <211> 19
   <212> DNA
   <213> Artifical Sequence
<220>
   <223> Description of Artificial Sequence: Synthetic DNA
<400> 22
   ggtgcaccga tgcaggggg 19
<210> 23
   <211> 14
   <212> DNA
   <213> Artifical Sequence
<220>
   <223> Description of Artificial Sequence: Synthetic DNA
<400> 23
   gtcgtcgacg acgg 14
<210> 24
   <211> 12
   <212> DNA
   <213> Artifical Sequence
<220>
   <223> Description of Artificial Sequence: Synthetic DNA
<400> 24
   gggggcgttg gg 12
<210> 25
   <211> 20
   <212> DNA
   <213> Artifical Sequence
<220>
   <223> Description of Artificial Sequence: Synthetic DNA
<400> 25
   atgcactctg cagcgttctc 20
<210> 26
   <211> 20
   <212> DNA
   <213> Artifical Sequence
<220>
   <223> Description of Artificial Sequence: Synthetic DNA
<400> 26
   atcgactctc gaggcttctc 20
<210> 27
   <211> 17
   <212> DNA
   <213> Artifical Sequence
<220>
   <223> Description of Artificial Sequence: Synthetic DNA
<400> 27
   ggtgcatcga tgcaggg 17
<210> 28
   <211> 25
   <212> DNA
   <213> Artifical Sequence
<220>
   <223> Description of Artificial Sequence: Synthetic DNA
<400> 28
   gggtcgtcgt tttgtcgttt cgttg 25
<210> 29
   <211> 12
   <212> DNA
   <213> Artifical Sequence
<220>
   <223> Description of Artificial Sequence: Synthetic DNA
<400> 29
   aaaggcgtta aa 12
<210> 30
   <211> 12
   <212> DNA
   <213> Artifical Sequence
<220>
   <223> Description of Artificial Sequence: Synthetic DNA
<400> 30
   cccggcgttc cc 12
<210> 31
   <211> 12
   <212> DNA
   <213> Artifical Sequence
<220>
   <223> Description of Artificial Sequence: Synthetic DNA
<400> 31
   gtcatcgatg ca 12
<210> 32
   <211> 20
   <212> DNA
   <213> Artifical Sequence
<220>
   <223> Description of Artificial Sequence: Synthetic DNA
<400> 32
   ggtgcatcga tgcagggggg 20
<210> 33
   <211> 20
   <212> DNA
   <213> Artifical Sequence
<220>
   <223> Description of Artificial Sequence: Synthetic DNA
<400> 33
   ggggtcatcg atgaaaaaaa 20
<210> 34
   <211> 20
   <212> DNA
   <213> Artifical Sequence
<220>
   <223> Description of Artificial Sequence: Synthetic DNA
<400> 34
   ggtgcatcga tgcagggggg 20
<210> 35
   <211> 20
   <212> DNA
   <213> Artifical Sequence
<220>
   <223> Description of Artificial Sequence: Synthetic DNA
<400> 35
   aaggtcaacg ttgaaaaaaa 20
<210> 36
   <211> 20
   <212> DNA
   <213> Artifical Sequence
<220>
   <223> Description of Artificial Sequence: Synthetic DNA
<400> 36
   aaggtcatcg atgggggggg 20
<210> 37
   <211> 20
   <212> DNA
   <213> Artifical Sequence
<220>
   <223> Description of Artificial Sequence: Synthetic DNA
<400> 37
   ggtgcatcga tgcagggggg 20
<210> 38
   <211> 20
   <212> DNA
   <213> Artifical Sequence
<220>
   <223> Description of Artificial Sequence: Synthetic DNA
<400> 38
   ggtgcatcga tgcagggggg 20
<210> 39
   <211> 20
   <212> DNA
   <213> Artifical Sequence
<220>
   <223> Description of Artificial Sequence: Synthetic DNA
<400> 39
   ggtgcgtcga cgcagggggg 20
<210> 40
   <211> 20
   <212> DNA
   <213> Artifical Sequence
<220>
   <223> Description of Artificial Sequence: Synthetic DNA
<400> 40
   ggtgcgtcga tgcagggggg 20
<210> 41
   <211> 20
   <212> DNA
   <213> Artifical Sequence
<220>
   <223> Description of Artificial Sequence: Synthetic DNA
<400> 41
   ggtgcgtcga cgcagggggg 20
<210> 42
   <211> 20
   <212> DNA
   <213> Artifical Sequence
<220>
   <223> Description of Artificial Sequence: Synthetic DNA
<400> 42
   ggtgcaccgg tgcagggggg 20
<210> 43
   <211> 20
   <212> DNA
   <213> Artifical Sequence
<220>
   <223> Description of Artificial Sequence: Synthetic DNA
<400> 43
   ggtgcatcga tgcagggggg 20
<210> 44
   <211> 12
   <212> DNA
   <213> Artifical Sequence
<220>
   <223> Description of Artificial Sequence: Synthetic DNA
<400> 44
   gtcaacgtcg ac 12
<210> 45
   <211> 12
   <212> DNA
   <213> Artifical Sequence
<220>
   <223> Description of Artificial Sequence: Synthetic DNA
<400> 45
   gtcggcgtcg ac 12
<210> 46
   <211> 19
   <212> DNA
   <213> Artifical Sequence
<220>
   <223> Description of Artificial Sequence: Synthetic DNA
<400> 46
   ggggtcaacg ttgaggggg 19
<210> 47
   <211> 12
   <212> DNA
   <213> Artifical Sequence
<220>
   <223> Description of Artificial Sequence: Synthetic DNA
<400> 47
   gtcggcgctg ac 12
<210> 48
   <211> 20
   <212> DNA
   <213> Artifical Sequence
<220>
   <223> Description of Artificial Sequence: Synthetic DNA
<400> 48
   atgcactctc gaggcttctc 20
<210> 49
   <211> 17
   <212> DNA
   <213> Artifical sequence
<220>
   <223> Description of Artificial Sequence: Synthetic DNA
<400> 49
   aatgcatcga tgcaaaa 17
<210> 50
   <211> 12
   <212> DNA
   <213> Artifical Sequence
<220>
   <223> Description of Artificial Sequence: Synthetic DNA
<400> 50
   gtcagcgtcg ac 12
<210> 51
   <211> 12
   <212> DNA
   <213> Artifical Sequence
<220>
   <223> Description of Artificial Sequence: Synthetic DNA
<400> 51
   gtcaacgttg ac 12
<210> 52
   <211> 12
   <212> DNA
   <213> Artifical Sequence
<220>
   <223> Description of Artificial Sequence: Synthetic DNA
<400> 52
   tgcatcgatg ca 12
<210> 53
   <211> 19
   <212> DNA
   <213> Artifical Sequence
<220>
   <223> Description of Artificial Sequence: Synthetic DNA
<400> 53
   ggtgcatcga tgcaggggg 19
<210> 54
   <211> 12
   <212> DNA
   <213> Artifical Sequence
<220>
   <223> Description of Artificial Sequence: Synthetic DNA
<400> 54
   gtcgacgtcg ac 12
<210> 55
   <211> 12
   <212> DNA
   <213> Artifical Sequence
<220>
   <223> Description of Artificial Sequence: Synthetic DNA
<400> 55
   gtcgacgccg ac 12
<210> 56
   <211> 12
   <212> DNA
   <213> Artifical Sequence
<220>
   <223> Description of Artificial Sequence: Synthetic DNA
<400> 56
   cccaacgttc cc 12
<210> 57
   <211> 12
   <212> DNA
   <213> Artifical Sequence
<220>
   <223> Description of Artificial Sequence: Synthetic DNA
<400> 57
   gtcaacgctg ac 12
<210> 58
   <211> 10
   <212> DNA
   <213> Artifical Sequence
<220>
   <223> Description of Artificial Sequence: Synthetic DNA
<400> 58
   gagcgttctc 10
<210> 59
   <211> 12
   <212> DNA
   <213> Artifical Sequence
<220>
   <223> Description of Artificial Sequence: Synthetic DNA
<400> 59
   gggaacgttg gg 12
<210> 60
   <211> 12
   <212> DNA
   <213> Artifical Sequence
<220>
   <223> Description of Artificial Sequence: Synthetic DNA
<400> 60
   gtcagcgctg ac 12
<210> 61
   <211> 16
   <212> DNA
   <213> Artifical Sequence
<220>
   <223> Description of Artificial Sequence: Synthetic DNA
<400> 61
   gggggaacgt tcgggg 16
<210> 62
   <211> 12
   <212> DNA
   <213> Artifical Sequence
<220>
   <223> Description of Artificial Sequence: Synthetic DNA
<400> 62
   gtcggcgccg ac 12
<210> 63
   <211> 16
   <212> DNA
   <213> Artifical Sequence
<220>
   <223> Description of Artificial Sequence: Synthetic DNA
<400> 63
   ggggtaacgt tagggg 16
<210> 64
   <211> 12
   <212> DNA
   <213> Artifical Sequence
<220>
   <223> Description of Artificial Sequence: Synthetic DNA
<400> 64
   gtcaacgccg ac 12
<210> 65
   <211> 12
   <212> DNA
   <213> Artifical Sequence
<220>
   <223> Description of Artificial Sequence: Synthetic DNA
<400> 65
   tgcctcgagg ca 12
<210> 66
   <211> 12
   <212> DNA
   <213> Artifical Sequence
<220>
   <223> Description of Artificial Sequence: Synthetic DNA
<400> 66
   tttaacgttt tt 12
<210> 67
   <211> 12
   <212> DNA
   <213> Artifical Sequence
<220>
   <223> Description of Artificial Sequence: Synthetic DNA
<400> 67
   aaaaacgtta aa 12
<210> 68
   <211> 16
   <212> DNA
   <213> Artifical Sequence
<220>
   <223> Description of Artificial Sequence: Synthetic DNA
<400> 68
   gggggaagct tcgggg 16
<210> 69
   <211> 12
   <212> DNA
   <213> Artifical Sequence
<220>
   <223> Description of Artificial Sequence: Synthetic DNA
<400> 69
   gtcagcgccg ac 12
<210> 70
   <211> 11
   <212> DNA
   <213> Artifical Sequence
<220>
   <223> Description of Artificial Sequence: Synthetic DNA
<400> 70
   cgagcgttct c 11
<210> 71
   <211> 16
   <212> DNA
   <213> Artifical Sequence
<220>
   <223> Description of Artificial Sequence: Synthetic DNA
<400> 71
   ggtgcatcga tgcagg 16
<210> 72
   <211> 20
   <212> DNA
   <213> Artifical Sequence
<220>
   <223> Description of Artificial Sequence: Synthetic DNA
<400> 72
   ggtgcatcga tgcagggggg 20
<210> 73
   <211> 19
   <212> DNA
   <213> Artifical Sequence
<220>
   <223> Description of Artificial Sequence: Synthetic DNA
<400> 73
   ggtgcatcga tgcaggggg 19
<210> 74
   <211> 13
   <212> DNA
   <213> Artifical Sequence
<220>
   <223> Description of Artificial Sequence: Synthetic DNA
<400> 74
   ggcgtcgacg ggg 13
<210> 75
   <211> 19
   <212> DNA
   <213> Artifical Sequence
<220>
   <223> Description of Artificial Sequence: Synthetic DNA
<400> 75
   ggtgcgtcgt tgcaggggg 19
<210> 76
   <211> 19
   <212> DNA
   <213> Artifical Sequence
<220>
   <223> Description of Artificial Sequence: Synthetic DNA
<400> 76
   ggtgcgccga tgcaggggg 19
<210> 77
   <211> 16
   <212> DNA
   <213> Artifical Sequence
<220>
   <223> Description of Artificial Sequence: Synthetic DNA
<400> 77
   gggggatcga tcgggg 16
<210> 78
   <211> 13
   <212> DNA
   <213> Artifical Sequence
<220>
   <223> Description of Artificial Sequence: Synthetic DNA
<400> 78
   ggggtcgaca ggg 13
<210> 79
   <211> 19
   <212> DNA
   <213> Artifical Sequence
<220>
   <223> Description of Artificial Sequence: Synthetic DNA
<400> 79
   ggtgcgtcgg tgcaggggg 19
<210> 80
   <211> 16
   <212> DNA
   <213> Artifical Sequence
<220>
   <223> Description of Artificial Sequence: Synthetic DNA
<400> 80
   gggggatgca tcgggg 16
<210> 81
   <211> 20
   <212> DNA
   <213> Artifical Sequence
<220>
   <223> Description of Artificial Sequence: Synthetic DNA
<400> 81
   ggtgcgtcga tgcagggggg 20
<210> 82
   <211> 20
   <212> DNA
   <213> Artifical Sequence
<220>
   <223> Description of Artificial Sequence: Synthetic DNA
<400> 82
   ggtgcgtcga tgcagggggg 20
<210> 83
   <211> 19
   <212 DNA
   <213> Artifical Sequence
<220>
   <223> Description of Artificial Sequence: Synthetic DNA
<400> 83
   ggtgcgtcga tgcaggggg 19
<210> 84
   <211> 19
   <212> DNA
   <213> Artifical Sequence
<220>
   <223> Description of Artificial Sequence: Synthetic DNA
<400> 84
   ggtgcctcga ggcaggggg 19
<210> 85
   <211> 16
   <212> DNA
   <213> Artifical Sequence
<220>
   <223> Description of Artificial Sequence: Synthetic DNA
<400> 85
   gggggctcga gagggg 16
<210> 86
   <211> 16
   <212> DNA
   <213> Artifical Sequence
<220>
   <223> Description of Artificial Sequence: Synthetic DNA
<400> 86
   ggggtatcga tagggg 16
<210> 87
   <211> 19
   <212> DNA
   <213> Artifical Sequence
<220>
   <223> Description of Artificial Sequence: Synthetic DNA
<400> 87
   ggtgcatcga tgcgagaga 19
<210> 88
   <211> 19
   <212> DNA
   <213> Artifical Sequence
<220>
   <223> Description of Artificial Sequence: Synthetic DNA
<400> 88
   ggtgcatcga cgcaggggg 19
<210> 89
   <211> 20
   <212> DNA
   <213> Artifical Sequence
<220>
   <223> Description of Artificial Sequence: Synthetic DNA
<400> 89
   ggggtcaacg ttgagggggg 20
<210> 90
   <211> 20
   <212> DNA
   <213> Artifical Sequence
<220>
   <223> Description of Artificial Sequence: Synthetic DNA
<400> 90
   ggtgcatgca tgcagggggg 20
<210> 91
   <211> 20
   <212> DNA
   <213> Artifical Sequence
<220>
   <223> Description of Artificial Sequence: Synthetic DNA
<400> 91
   ggggtcaagc ttgagggggg 20
<210> 92
   <211> 16
   <212> DNA
   <213> Artifical Sequence
<220>
   <223> Description of Artificial Sequence: Synthetic DNA
<400> 92
   ggggtaagct tagggg 16
<210> 93
   <211> 17
   <212> DNA
   <213> Artifical Sequence
<220>
   <223> Description of Artificial Sequence: Synthetic DNA
<400> 93
   ggtgcatgca tgcaggg 17
<210> 94
   <211> 20
   <212> DNA
   <213> Artifical Sequence
<220>
   <223> Description of Artificial Sequence: Synthetic DNA
<400> 94
   ggtgcataaa tgcagggggg 20
<210> 95
   <211> 17
   <212> DNA
   <213> Artifical Sequence
<220>
   <223> Description of Artificial Sequence: Synthetic DNA
<400> 95
   aatgcatgca tgcaaaa 17
<210> 96
   <211> 20
   <212> DNA
   <213> Artifical Sequence
<220>
   <223> Description of Artificial Sequence: Synthetic DNA
<400> 96
   ggtgcatgca tgcagggggg 20
<210> 97
   <211> 20
   <212> DNA
   <213> Artifical Sequence
<220>
   <223> Description of Artificial Sequence: Synthetic DNA
<400> 97
   atcgactctg caggcttctc 20
<210> 98
   <211> 12
   <212> DNA
   <213> Artifical Sequence
<220>
   <223> Description of Artificial Sequence: Synthetic DNA
<400> 98
   tcgaggcttc tc 12
<210> 99
   <211> 20
   <212> DNA
   <213> Artifical Sequence
<220>
   <223> Description of Artificial Sequence: Synthetic DNA
<400> 99
   atgcactctg caggcttctc 20
<210> 100
   <211> 20
   <212> DNA
   <213> Artifical Sequence
<220>
   <223> Description of Artificial Sequence: synthetic DNA
<400> 100
   ggtgcatcga cgcagggggg 20
<210> 101
   <211> 20
   <212> DNA
   <213> Artifical Sequence
<220>
   <223> Description of Artificial Sequence: Synthetic DNA
<400> 101
   ggtgcaccga tgcagggggg 20
<210> 102
   <211> 20
   <212> DNA
   <213> Artifical Sequence
<220>
   <223> Description of Artificial Sequence: Synthetic DNA
<400> 102
   ggtgtgtcga tgcagggggg 20
<210> 103
   <211> 20
   <212> DNA
   <213> Artifical Sequence
<220>
   <223> Description of Artificial Sequence: Synthetic DNA
<400> 103
   ggtgcaccgt ggcagggggg 20
<210> 104
   <211> 20
   <212> DNA
   <213> Artifical Sequence
<220>
   <223> Description of Artificial Sequence: Synthetic DNA
<400> 104
   ggtgcatcgt tgcagggggg 20
<210> 105
   <211> 12
   <212> DNA
   <213> Artifical Sequence
<220>
   <223> Description of Artificial Sequence: Synthetic DNA
<400> 105
   tcgtttgttc tc 12
<210> 106
   <211> 20
   <212> DNA
   <213> Artifical Sequence
<220>
   <223> Description of Artificial Sequence: Synthetic DNA
<400> 106
   ggtgcatcga tacagggggg 20
<210> 107
   <211> 20
   <212> DNA
   <213> Artifical Sequence
<220>
   <223> Description of Artificial Sequence: Synthetic DNA
<400> 107
   ggtgcattga tgcagggggg 20
<210> 108
   <211> 12
   <212> DNA
   <213> Artifical Sequence
<220>
   <223> Description of Artificial Sequence: Synthetic DNA
<400> 108
   tcgttcgttc tc 12
<210> 109
   <211> 14
   <212> DNA
   <213> Artifical Sequence
<220>
   <223> Description of Artificial Sequence: Synthetic DNA
<400> 109
   actctttcgt tctc 14
<210> 110
   <211> 14
   <212> DNA
   <213> Artifical Sequence
<220>
   <223> Description of Artificial Sequence: Synthetic DNA
<400> 110
   actctttcga tctc 14
<210> 111
   <211> 12
   <212> DNA
   <213> Artifical Sequence
<220>
   <223> Description of Artificial Sequence: Synthetic DNA
<400> 111
   tgcaggcttc tc 12
<210> 112
   <211> 16
   <212> DNA
   <213> Artifical Sequence
<220>
   <223> Description of Artificial Sequence: Synthetic DNA
<400> 112
   actcttgagt gttctc 16

## Description

### TECHNICAL FIELD OF THE INVENTION

The present invention pertains generally to induction of an immune response using different CpG sequences.

### BACKGROUND OF THE INVENTION

DNA is a complex macromolecule whose immunological activities are influenced by its base composition and base modification, as well as helical orientation. Certain unusual DNA structures (e.g., Z-DNA) can induce significant antibody responses when administered to normal mice. In addition, bacterial DNA, as well as certain synthetic unmethylated CpG sequences can induce proliferation and immunoglobulin (Ig) production by murine B cells. Unmethylated CpG dinucleotides are more frequent in the genomes of bacteria and viruses than vertebrates, and recent studies suggest that immune recognition of these motifs may contribute to the host's innate immune response. D.M. Klinman et al., CpG Motifs Present in Bacterial DNA Rapidly Induce Lymphocytes to Secrete Interleukin 6, Interleukin 12, and Interferon y, 93 Proc. Natl. Acad. Sci. USA 2879 (1996); A.-K. Yi et al., Rapid Immune Activation by CpG Motifs in Bacterial DNA, 157 J. Immun. 5394 (1996); Hua Liang et al., Activation of Human B Cells by Phosphorothioate Oligodeoxynucleotides, 98 J. Clin. Invest. 1119 (1996); A.M. Krieg et al., CpG Motifs in Bacterial DNA Trigger Direct B-Cell Activation, 374 Nature 546 (1995).

In mice, CpG DNA induces proliferation in almost all (>95%) of B cells and increases Ig secretion. This B cell activation by CpG DNA is T-cell independent and antigen non-specific. In addition to its direct effects on B cells, CpG DNA also directly activates monocytes, macrophages, and dendritic cells to secrete a variety of cytokines. These cytokines stimulate natural killer (NK) cells to secrete γ-interferon (IFN-γ) and have increased lytic activity. Examples of which can be found in International Patent Applications WO 95/26204, WO 96/02555, WO 98/11211; WO 98/18810, WO 98/37919, WO 98/40100, WO 98/52581; U.S. Patent Application Nos. 08/738,652; and U.S. Patent No. 5,663,153.

Although bacterial DNA and certain CpG sequences can induce responses from human cells (Z.K. Ballas et al., Induction of NK Activity in Murine and Human Cells by CpG Motifs in Oligodeoxynucleotides and Bacterial DNA, 157 J. Immunol. 1840 (1996)), individual subjects show considerable heterogeneity in their response to different CpG sequences. Indeed, CpG sequences that strongly stimulate cells from some subjects are virtually inactive on cells from other subjects. These different responses make it difficult, it not impossible, to induce a therapeutic immune response in all members of a diverse population using a single CpG sequence, even if such a sequence is expressed repetitively in a given oligonucleotide.

In view of the above, there exists a need to identity different CpG sequences that together are capable of optimally inducing an immune response in cells from all members of a target population. In addition, there is a need for methods utilizing these CpG sequences in the treatment of diseases. The present invention provides such CpG sequences and methods of use. These and other advantages of the present invention, as well as additional inventive features, will be apparent from the description of the invention provided herein.

### BRIEF SUMMARY OF THE INVENTION

The present invention provides a substantially pure or isolated oligodeoxynucleotide (ODN) of at least about 10 nucleotides comprising multiple CpG sequences, wherein at least one of the CpG sequences is different from another of the multiple CpG sequences as recited in claim 1. The present invention also provides an ODN delivery complex and a pharmacological composition comprising an ODN or ODNs, as well as their use in a method of inducing an immune response by administering an ODN or ODNs to a host as recited in the claims.

### DETAILED DESCRIPTION OF THE INVENTION

The present invention is based on the discovery that different CpG sequences, which are formulated either as multiple individual oligodeoxynucleotides (ODNs) each comprising a single CpG motif, or a complex ODN comprising multiple CpG sequences, induces an enhanced immune response in a broad population.

### Oligodeoxynucleotide

The present invention provides novel ODNs. These ODNs have at least about 10 nucleotides and comprise multiple (i.e., 2 or more) CpG sequences, wherein at least one of the multiple CpG sequences is different from another of the multiple CpG sequences. A "CpG sequence" or "CpG motif" refers to a nucleic acid sequence having a cytosine followed by a guanine linked by a phosphate bond in which the cytosine is unmethylated.

The ODNs of the present invention comprise multiple different CpG sequences as set forth in claim 1. In addition, CpG sequences selected from the group consisting of SEQ ID NO:1 through SEQ ID NO:112 may be used.

Preferably, the ODN of the present invention is substantially pure or isolated. "Substantially pure" refers to an ODN that is substantially free of other materials, particularly other nucleic acids, proteins, lipids, carbohydrates, and other materials with which it may be naturally associated, while "isolated" refers to an ODN that is removed from its natural environment or state. The ODN of the present invention can consist of about 50 nucleotides or less (e.g., about 10-40 nucleotides).

Preferably, the ODNs inducing a humoral immune response, e.g., those containing a sequence as set forth in claim 1 contain a phosphate backbone modification, and more preferably, the phosphate backbone modification is a phosphorothioate backbone modification (i.e., one of the non-bridging oxygens is replaced with sulfur, as set forth in International Patent Application WO 95/26204). For the ODNs of claim 1 inducing a cell-mediated immune response and containing a phosphodiester backbone, the ODN preferably has been modified to prevent degradation.

Any suitable modification can be used in the present invention to render the ODN resistant to *in vivo* degradation resulting from, e.g., exo or endonuclease digestion. Preferably, the modification includes a phosphorothioate modification. The phosphorothioate modifications can occur at either termini, e.g., the last two or three 5' and/or 3' nucleotides can be liked with phosphorothioate bonds. The ODN also can be modified to contain a secondary structure (e.g., stem loop structure) such that it is resistant to degradation. Another modification that renders the ODN less susceptible to degradation is the inclusion of nontraditional bases such as inosine and quesine, as well as acetyl-, thio- and similarly modified forms of adenine, cytidine, guanine, thymine, and uridine. Other modified nucleotides include nonionic DNA analogs, such as alkyl or aryl phosphonates (i.e., the charged phosphonate oxygen is replaced with an alkyl or aryl group, as set forth in U.S. Patent No. 4,469,863), phosphodiesters and alkylphosphotriesters (i.e., the charged oxygen moiety is alkylated, as set forth in U.S. Patent No. 5,023,243 and European Patent No. 0 092 574). ODNs containing a diol, such as tetraethyleneglycol or hexaethyleneglycol, at either or both termini, have also been shown to be more resistant to degradation.

### Oligodeoxynucleotide Delivery Complex

The present inventive ODN delivery complex can comprise multiple (i.e.. more than one) substantially pure or isolated ODNs of at least about 10 nucleotides comprising a CpG sequence as set forth in claim 2 and a targeting means, wherein at least one of the CpG sequences is different from another of the multiple CpG sequences. Therefore, the present inventive ODN delivery complex can comprise multiple ODNs comprising a single CpG sequence with at least one of these multiple ODNs comprising a CpG sequence that is different from the CpG sequences comprised by another ODN within the complex.

Additionally, the present inventive ODN delivery complex can comprise a single ODN or multiple ODNs comprising multiple different CpG sequences and a targeting means. Therefore, the present inventive ODN delivery complex can comprise either a single ODN, or multiple (i.e., more than one) ODNs.

Any suitable targeting means (i.e., a molecule that results in higher affinity binding to a target cell) can be used within the context of the present invention. Suitable targeting means are well known in the art. The ODN delivery complex can be associated with (e.g., ionically or covalently bound to, or encapsulated within) the targeting means by a variety of coupling or cross-linking agents, e.g., protein A, carbodiamide, and N-succinimidyl-3-(2-pyridyldithio) propionate (SPDP). Examples of ODN delivery complexes include ODNs associated with a sterol (e.g., cholesterol), a lipid (e.g., a cationic lipid, virosome or liposome), and a target cell specific binding agent (e.g., a ligand recognized by target cell specific receptor). Preferred complexes must be sufficiently stable *in vivo* to prevent significant uncoupling prior to internalization by the target cell; however, these complexes can be cleavable under appropriate circumstances such that the ODN can be released in a functional form.

### Pharmacological Composition

The present inventive composition can comprise multiple substantially pure or isolated ODNs of at least about 10 nucleotides comprising a CpG sequence and a pharmacologically acceptable carrier, wherein at least one of the CpG sequences is different from another of the multiple CpG sequences. Therefore, the present inventive pharmacological composition can comprise multiple ODNs comprising a single CpG sequence and at least one of these multiple ODNs comprises a CpG sequence that is different from the CpG sequences comprised by another ODN within the composition.

Additionally, the present inventive pharmaceutical composition can comprise a single ODN or multiple ODNs comprising multiple different CpG sequences and a pharmacologically acceptable carrier. Therefore, the present inventive pharmaceutical composition can comprise either a single ODN, or multiple (i.e., more than one) ODNs.

Pharmacologically acceptable carriers (e.g., physiologically or pharmaceutically acceptable carriers) are well known in the art. The present inventive pharmacological composition facilitates the use of the one or more present inventive ODNs, both *in vivo* and *ex vivo.* Such a composition can be suitable for delivery of the active ingredient to any suitable host, such as a patient for medical application, and can be manufactured in a manner that is itself known, e.g., by means of conventional mixing, dissolving, granulating, dragee-making, levigating, emulsifying, encapsulating, entrapping or lyophilizing processes.

Pharmacological compositions for use in accordance with the present invention can be formulated in a conventional manner using one or more pharmacologically (e.g., physiologically or pharmaceutically) acceptable carriers comprising excipients, as well as optional auxiliaries that facilitate processing of the active compounds into preparations that can be used pharmaceutically. Proper formulation is dependent upon the route of administration chosen. Thus, for injection, the active ingredient can be formulated in aqueous solutions, preferably in physiologically compatible buffers. For transmucosal administration, penetrants appropriate to the barrier to be permeated are used in the formulation. Such penetrants are generally known in the art. For oral administration, the active ingredient can be combined with carriers suitable for inclusion into tablets, pills, dragees, capsules, liquids, gels, syrups, slurries, suspensions and the like. For administration by inhalation, the active ingredient is conveniently delivered in the form of an aerosol spray presentation from pressurized packs or a nebuliser, with the use of a suitable propellant. The active ingredient can be formulated for parenteral administration by injection, e.g., by bolus injection or continuous infusion. Such compositions can take such forms as suspensions, solutions or emulsions in oily or aqueous vehicles, and can contain formulatory agents such as suspending, stabilizing and/or dispersing agents. Other pharmacological excipients are known in the art.

### Method of Inducing an Immune Response

The present inventive use of ODN or ODNs in a method of inducing an immune response can comprise administering to a host multiple substantially pure or isolated ODNs of at least about 10 nucleotides comprising a CpG sequence in order to induce an immune response in the host, wherein at least one of the CpG sequences is different from another of the multiple CpG sequences. Therefore, the present method can comprise administering to a host multiple ODNs comprising a single CpG sequence and at least one of these multiple ODNs comprises a CpG sequence that is different from the CpG sequences comprised by another ODN administered to a host.

Additionally, the present inventive use of ODN or ODNs in a method of inducing an immune response can comprise administering to a host a single ODN or multiple ODNs comprising multiple different CpG sequences in order to induce an immune response in the host. Therefore, the present inventive method of inducing an immune response can comprise administering either a single ODN or multiple (i.e., more than one) ODNs to a host in order to induce an immune response in the host.

Administration can be by any suitable method. For example, the ODN or ODNs can be administered *in vivo* or *ex vivo.* Preferably, the ODN or ODNs are administered *in vivo* to a mammal, particularly a human. Optionally, the ODN or ODNs can be contained within or conjugated with a larger nucleic acid molecule, protein, hydrocarbon or lipid. Once this molecule is administered, the CpG sequences must be exposed on the surface to induce an immune response. Examples of suitable nucleic acid molecules include fusion or chimeric nucleic acids, proteins, hydrocarbons and lipids. The ODN or ODNs can also be co-administered with another nucleic acid, protein, hydrocarbon, or lipid. Co-administration can be such that the ODN or ODNs are administered before, at substantially the same time as, or after the other nucleic acid, protein, hydrocarbon. or lipid. Preferably, the ODN or ODNs are administered at substantially the same time as the other nucleic acid, protein, hydrocarbon, or lipid.

After administration of the ODN or ODNs, while not intending to be bound by any particular theory, it is thought that the ODN initially acts on antigen presenting cells (e.g., macrophages and dendritic cells). These cells then release cytokines, which activate natural killer (NK) cells. Either a cell-mediated or humoral immune response then occurs in the host.

The cell-mediated or local immune response is produced by T cells, which are able to detect the presence of invading pathogens through a recognition system referred to as the T cell antigen receptor. Upon detection of an antigen, T cells direct the release of multiple T cell cytokines, including IL-2, IL-3, IFN-γ, TNF-β, GM-CSF and high levels of TNF-α, and chemokines MIP-1α, MIP-1β, and RANTES. IL-2 is a T cell growth factor that promotes the production of additional T cells sensitive to the particular antigen. This production constitutes a clone of the T cells. The sensitized T cells attach to cells containing the antigen. T cells carry out a variety of regulatory and defense functions and play a central role in immunologic responses. When stimulated to produce a cell-mediated immune response, some T cells respond by acting as killer cells, killing the host's own cells when these cells are infected or cancerous and therefore recognized as foreign. Some T cells respond by stimulating B cells, while other T cells respond by suppressing immune response. If a cell-mediated immune response is induced, preferably, non-B cells are activated, more preferably, cytokines are produced, and most preferably, IFN-γ is produced.

The humoral or systemic immune response depends on the ability of the B cells to recognize specific antigens. The mechanism by which B cells recognize antigens is through specific receptors on the surface of the B cells. When an antigen attaches to the receptor site of a B cell, the B cell is stimulated to divide. The daughter cells become plasma cells that manufacture antibodies complementary to the attached antigen. Each plasma cell produces thousands of antibody molecules per minute, which are released into the bloodstream. Many B cells appear to be regulated by the helper T cells and suppressor T cells and produce various cytokines, e.g., IL-3, IL-4, IL-5, IL-6, IL-9, IL-10, IL-13, GM-CSF and low levels of TNF-α. Helper T cells stimulate B cells to produce antibodies against antigens, while suppressor T cells inhibit antibody production. Some B cells, however, are T cell independent and require no stimulation by the T cells. If a humoral immune response is induced, preferably, B cells are activated, more preferably, IL-6 is produced, and most preferably, antibodies are produced.

In addition, induction of one type of immune response may allow for immune regulation because up regulation of one type of immune response may down regulate the other type of immune response. This immune regulation allows for customizing or tailoring of the type of immune response when administering an ODN.

The present inventive method of inducing an immune response can be used to treat, prevent, or ameliorate any suitable allergic reaction. Optionally, the present inventive method can be used in combination with any suitable anti-allergenic agent. An allergy, in the context of the present invention, refers to an acquired hypersensitivity to a substance (i.e., an allergen). Allergic conditions include eczema, allergic rhinitis or coryza, hay fever, bronchial asthma, uticaria (hives), food allergies, and other atopic conditions. The list of allergens is extensive and includes pollens, insect venoms, animal dander, dust, fungal spores, and drugs (e.g., penicillin). Examples of natural, animal, and plant allergens can be found in International Patent Application WO 98/18810. Preferably, the present inventive method is used to treat allergic asthma. Suitable anti-allergenic agents include those substances given in treatment of the various allergic conditions described above, examples of which can be found in the Physicians' Desk Reference (1998).

The present inventive method of inducing an immune response can be used to treat any suitable cancer. Optionally, the present inventive method can be used in combination with any suitable anti-cancer agent. Suitable cancers include cancers of the brain, lung (e.g., small cell and non-small cell), ovary, breast, prostate, and colon, as well as carcinomas and sarcomas. Preferably, the present inventive method is used to treat a solid tumor cancer. Suitable anti-cancer agents include those substances given in treatment of the various conditions described above, examples of which can be found in the Physicians' Desk Reference (1998).

The present inventive method of inducing an immune response can be used to improve the efficacy of any suitable vaccine. Suitable vaccines include those directed against Hepatitis A, B, and C, examples of which can be found in the Physicians' Desk Reference (1998), and DNA vaccines directed against HIV and malaria. *See generally* D. Klinman et al., CpG Motifs as Immune Adjuvants, 17 Vaccine 19 (1999); M.J. McCluskie and H.L. Davis, CpG DNA is a Potent Enhancer of Systemic & Mucosal Immune Response Against Hepatitis B Surface Antigen with Intra-Nasal Administration to Mice, 161 J. Immun. 4463 (1998).

The present inventive method of inducing an immune response can be used to treat, prevent, or ameliorate any suitable disease associated with the immune system. Preferred diseases associated with the immune system are autoimmune disorders and immune system deficiencies, e.g., lupus erythematosus, and autoimmune diseases such as rheumatoid arthritis and multiple sclerosis. Immune system deficiencies include those diseases or disorders in which the immune system is not functioning at normal capacity, or in which it would be useful to boost the immune system response.

The present inventive method of inducing an immune response can be used with any suitable antisense therapy. Optionally, the present inventive method can be used in combination any suitable antisense agent. Suitable antisense agents are those that bind either with DNA or RNA and block their function by inhibiting expression of the sequence to which the antisense agents are bound. *See generally* H. Lonnberg et al., Towards Genomic Drug Therapy with Antisense Oligonucleotides, 28 Ann. Med. 511 (1996); A. Alama et al., Antisense Oligonucleotidesas Therapeutic Agents, 36 Pharmacol. Res. 171 (1997); K.J. Scanlon et al., Oligonucleotide-Mediated Modulation of Mammalian Gene Expression, 9 FASEB J. 1288 (1995); R. Oberbauer, Not Non-Sense but Antisense -- Applications of Antisense Oligonucleotides in Different Fields of Medicine, 109 Wien Klin Wochenschr 40 (1997).

The present inventive method of inducing an immune response can be used to treat, prevent, or ameliorate any suitable infection. Optionally, the present inventive method can be used in combination with any suitable anti-infectious agent. Examples of infections include francisella, schistosomiasis, tuberculosis, AIDS, malaria, and leishmania. Examples of suitable infectious viruses, bacteria, fungi, and other organisms (e.g., protists) can be found in International Patent Application WO 98/18810. Suitable anti-infectious agents include those substances given in treatment of the various conditions described elsewhere, examples of which can be found in the Physicians' Desk Reference (1998).

The present inventive method of inducing an immune response can be used to treat, prevent, or ameliorate the symptoms resulting from exposure to a bio-warfare agent. Suitable bio-warfare agents include those naturally occurring biological agents that have been specifically modified in the laboratory. Often, modification of these agents has altered them such that there is no known treatment. Examples include Ebola, Anthrax, and Listeria. In the course of ameliorating the symptoms after exposure, use of the present inventive ODNs may not cure the patient, but rather can extend the patient's life sufficiently such that some other treatment can then be applied.

The present invention is further described in the following examples. These examples are intended only to illustrate the invention and are not intended to limit the scope of the invention in any way.

### EXAMPLES

### Example 1

The following example demonstrates the varied immune response induced *in vitro* in different donors after administration of an ODN comprising a single CpG sequence. Induction of an immune response was measured by production of the cytokines IL-6 and IFN-γ, and cell proliferation in human peripheral blood mononuclear cells (PBMC) isolated from individual donors.

PBMC were isolated, as described elsewhere (Z.K. Ballas et al., 85 J. Allergy Clin. Immunol. 453 (1990); Z.K. Ballas and W. Rasmussen, 45 J. Immunol. 1039 (1990); Z.K. Ballas and W. Rasmussen, 150 J. Immunol. 17 (1993)). ODNs were synthesized on a DNA synthesizer (Applied Biosystems Inc., Foster City, CA), as described elsewhere (Beacage and Caruthers, Deoxynucleoside Phosphoramidites - A New Class of Key Intermediates for Deoxypolynucleotide Synthesis, 22 Tetrahedron Letters 1859 (1981)). In some ODNs, the normal DNA backbone phosphodiesterase linkages were replaced with phosphorothioate linkages, as described elsewhere (Agrawal et al., 94 Proc. Natl. Acad. Sci. USA 2620 (1997); Agrawal 14 TIB TECH 376 (1996)). To reduce degradation of the ODNs, those that did not have an entire phosphorothioate backbone contained phosphorothioate linkages at the 5' and 3' ends. Cells from the different donors were incubated for approximately 72 hrs with the various ODNs. IL-6 and IFN-γ levels were determined by ELISA using anti-IL-6 and anti-IFN-γ antibodies, as described elsewhere (Maniatis et al.. Molecular Cloning: A Laboratory Manual, Cold Spring Harbor Laboratory Press, New York, 1989). Cell proliferation was determined by [³H] thymidine incorporation, as described elsewhere (Liang et al., 98 J. Clin. Invest. at 1121). IL-6 levels are set forth in Table 1: Induction of an Immune Response (IL-6), IFN-γ levels are set forth in Table 5: Induction of an Immune Response (IFN-γ), and cell proliferation is set forth in Table 6: Induction of an Immune Response (Cell Proliferation).

**TABLE 1: Induction of an Immune Response (IL-6)**

| | 3 | 4 | 7 | 8 | 15 | 16 | 17 | 18 | 19 | 23 | 24 | 25 | 26 | 27 |
|---|---|---|---|---|---|---|---|---|---|---|---|---|---|---|
| SEQ ID NO:1 | 35 | 53 | 19 | 12 | 9 | 2 | 8 | 6 | 33 | 15 | 5 | 40 | 13 | 3 |
| SEQ ID NO:18 | 2 | 3 | 25 | 65 | -- | -- | -- | -- | -- | 8 | 3 | 3 | 1 | 1 |
| SEQ ID NO:20 | 50 | 45 | 9 | 43 | 5 | 9 | 20 | 20 | 20 | -- | 12 | 57 | 13 | 3 |
| SEQ ID NO:98 | 18 | 42 | 9 | 41 | 60 | 80 | 25 | 11 | 22 | 17 | 6 | 12 | 2 | 1 |
| SEQ ID NO:105 | -- | -- | -- | -- | -- | -- | -- | -- | -- | 13 | 7 | 16 | 3 | 1 |

**TABLE 2: Induction of an Immune Response (IFN-γ)**

| | 3 | 4 | 5 | 9 | 13 | 14 | 15 | 17 |
|---|---|---|---|---|---|---|---|---|
| SEQ ID NO:40 | 154 | 64 | 6 | 2 | 5 | 11 | 15 | 3 |
| SEQ ID NO:42 | 92 | 56 | 419 | 28 | 8 | 4 | 4 | 8 |
| SEQ ID NO:43 | 13 | 3 | 269 | 93 | 15 | 6 | 5 | 8 |
| SEQ ID NO:100 | 22 | 2 | 16 | 2 | 9 | 9 | 9 | 6 |
| SEQ ID NO:101 | 3 | 2 | 15 | 25 | 0 | 16 | 5 | 5 |
| SEQ ID NO:102 | -- | -- | 0 | 1 | 60 | 250 | 6 | 3 |
| SEQ ID NO:103 | -- | -- | 4 | 1 | 3 | 2 | 8 | 5 |
| SEQ ID NO:104 | -- | -- | 4 | 1 | 2 | 1 | 3 | 4 |

**TABLE 3: Induction of an Immune Response (Cell Proliferation),**

| | 1 | 2 | 3 | 4 | 20 | 21 | 22 |
|---|---|---|---|---|---|---|---|
| SEQ ID NO:1 | 22 | 36 | 33 | 9 | 18 | 15 | 14 |
| SEQ ID NO:9 | 5 | 17 | 16 | 11 | 18 | 18 | 13 |
| SEQ ID NO:10 | 9 | 10 | 20 | 25 | 14 | 23 | 12 |
| SEQ ID NO:12 | 6 | 8 | 10 | 19 | 4 | 7 | 6 |
| SEQ ID NO: 15 | 9 | 47 | 11 | 13 | 14 | 17 | 12 |
| SEQ ID NO:31 | 2 | 15 | 10 | 16 | 6 | 8 | 7 |
| SEQ ID NO:45 | 3 | 7 | 16 | 15 | 10 | 16 | 10 |
| SEQ ID NO:50 | 3 | 6 | 10 | 9 | 6 | 6 | 5 |
| SEQ ID NO:54 | 4 | 5 | 10 | 9 | 5 | 5 | 4 |

The foregoing data demonstrates induction of an immune response to an ODN comprising various sequences in human PBMC isolated from individual donors. Specifically, these data demonstrate that a single sequence induces a varied immune response in different donors, as show, e.g., in Table 4, an ODN comprising SEQ ID NO:98 induced IL-6 levels ranging from 2 to 80, in Table 5, an ODN comprising SEQ ID NO:42 induced IFN-γ levels ranging from 4 to 419, and in Table 6, an ODN comprising SEQ ID NO:15 induced cell proliferation ranging from 9 to 47.

### Example 2

The following example demonstrates the varied immune responses induced *in vitro* in different donors after administration of an ODN comprising a single CpG sequence. Induction of an immune response was measured by production of the cytokines IL-6 and IFN-γ in human PBMC isolated from individual donors as compared to unstimulated PBMC from the same donor.

Human PBMC were isolated, as described in Example 1. ODNs were synthesized on a DNA synthesizer (Applied Biosystems Inc., Foster City, CA), as described in Example 1. In some ODNs, the normal DNA backbone phosphodiesterase linkages were replaced with phosphorothioate linkages, as described in Example 1. To reduce degradation of the ODNs, those that did not have an entire phosphorothioate backbone contained phosphorothioate linkages at the 5' and 3' ends. Cells were incubated for approximately 72 hrs with the various ODNs. IL-6 and IFN-γ levels were determined by ELISA using anti-IL-6 and anti-IFN-γ antibodies, as described in Example 1. The percentage of donors induced by the various ODNs at least 3-fold for IL-6 and 5-fold for IFN-γ are set forth for IL-6 levels in Table 4: Percent Induction of an Immune Response (IL-6) and for IFN-γ levels in Table 5: Induction of an Immune Response (IFN-γ). Also, a profile was created for some of the donors in which greater than a 10-fold increase is represented by "++", greater than a 3-fold increase for IL-6 and greater than a 5-fold increase for IFN-γ is represented by "+"and any levels lower than this are represented by "--". These profiles are set forth for IL-6 in Table 6: Heterogeneity in Induction of an Immune Response (IL-6) and for IFN-γ in Table 7: Heterogeneity in Induction of an Immune Response (IFN-γ).

**TABLE 4: Percent Induction of an Immune Response (IL-6)**

| | Percent Induction | Number of Donors |
|---|---|---|
| SEQ ID NO:1 | 69% | 26 |
| SEQ ID NO:109 | 67% | 9 |
| SEQ ID NO:20 | 65% | 34 |
| SEQ ID NO:7 | 49% | 39 |
| SEQ ID NO:108 | 47% | 38 |
| SEQ ID NO:98 | 44% | 39 |
| SEQ ID NO:110 | 30% | 10 |
| SEQ ID NO:111 | 26% | 19 |
| SEQ ID NO:112 | 7% | 29 |

**TABLE 5: Percent Induction of an Immune Response (IFN-γ)**

| | Percent Induction | Number of Donors |
|---|---|---|
| SEQ ID NO:43 | 93% | 42 |
| SEQ ID NO:42 | 91% | 23 |
| SEQ ID NO:106 | 87% | 23 |
| SEQ ID NO:32 | 82% | 17 |
| SEQ ID NO:40 | 79% | 19 |
| SEQ ID NO: 103 | 58% | 12 |
| SEQ ID NO:102 | 57% | 28 |
| SEQ ID NO:107 | 10% | 31 |
| SEQ ID NO:68 | 11% | 19 |

**TABLE 6: Heterogeneity in Induction of an Immune Response (IL-6)**

| | 1 | 2 | 3 | 4 | 5 | 6 | 7 | 8 |
|---|---|---|---|---|---|---|---|---|
| SEQ ID NO:20 | -- | ++ | + | + | + | + | ++ | ++ |
| SEQ ID NO:7 | - | + | + | -- | ++ | + | ++ | + |
| SEQ ID NO:1 | ++ | ++ | + | -- | + | + | ++ | -- |

**TABLE 7: Heterogeneity in Induction of an Immune Response (IFN-γ)**

| | 1 | 2 | 3 | 4 | 5 | 6 | 7 | 8 |
|---|---|---|---|---|---|---|---|---|
| SEQ ID NO:40 | ++ | ++ | -- | -- | -- | + | ++ | + |
| SEQ ID NO:32 | -- | ++ | + | + | ++ | + | -- | -- |
| SEQ ID NO:102 | -- | -- | ++ | ++ | ++ | + | -- | ++ |

The foregoing data demonstrates induction of an immune response to an ODN comprising various sequences in human PBMC isolated from individual donors. Specifically, these data demonstrate that a single sequence induces a varied immune response in different donors, as show, e.g., in Table 4, the percent of donors induced varied from 7% to 69%, as measured by IL-6 production, and in Table 5, the percent of donors induced varied from 11% to 93%, as measured by IFN-γ production. Further, as demonstrated in Tables 6 and 7, there was substantial heterogeneity in induction of an immune response in different donors.

### Example 3

The following example demonstrates *in vitro* induction of an immune response after administration of multiple ODNs comprising a CpG sequence. Induction of an immune response was measured by production of the cytokines IL-6 and IFN-γ, and cell proliferation in human PBMC isolated from individual donors.

Human PBMC were isolated, as described in Example 1. ODNs were synthesized on a DNA synthesizer (Applied Biosystems Inc., Foster City, CA), as described in Example 1. In some ODNs, the normal DNA backbone phosphodiesterase linkages were replaced with phosphorothioate linkages, as described in Example 1. To reduce degradation of the ODNs, those that did not have an entire phosphorothioate backbone contained phosphorothioate linkages at the 5' and 3' ends. Cells were incubated for approximately 72 hrs with the various ODNs. IL-6 and IFN-γ levels were determined by ELISA using anti-IL-6 and anti-IFN-γ antibodies, as described in Example 1. Cell proliferation was determined by [³H] thymidine incorporation, as described in Example 1. Results are set forth in Table 8: Induction of an Immune Response to Multiple ODNs.

**TABLE 8: Induction of an Immune Response to Multiple ODNs**

| | IL-6 (ELISA) | IFN-γ (ELISA) | Proliferation ([³H] T) |
|---|---|---|---|
| Donor 1 | | | |
| SEQ ID NO:1 | 12.0 | 2.3 | 13.0 |
| SEQ ID NO:43 | 7.0 | 9.0 | 1.6 |
| SEQ ID NO:1 + SEQ ID NO:43 | 27 | 40.0 | 19.9 |

| Donor 2 | | | |
|---|---|---|---|
| SEQ ID NO:1 | 5.0 | 13.0 | 37.7 |
| SEQ ID NO:43 | 2.0 | 7.0 | 1.3 |
| SEQ ID NO:1 + SEQ ID NO:43 | 9.0 | 33.0 | 68.6 |

| Donor 3 | | | |
|---|---|---|---|
| SEQ ID NO:1 | 10.0 | 8.0 | 13.1 |
| SEQ ID NO:43 | 1.0 | 12.0 | 1.3 |
| SEQ ID NO:1 + SEQ ID NO:43 | 8.0 | 42.0 | 17.5 |

The foregoing data demonstrates the induction of an immune response after administration of multiple ODNs comprising various CpG sequences in human PBMC isolated from individual donors. Specifically, these data demonstrate that multiple ODNs synergistically induce an immune response, as demonstrated by, e.g., an increase of 26.6% as measured by cell proliferation, 29.6% as measured by IL-6 levels and 71.7% as measured by IFN-γ levels after administration to Donor 1 of a combination of an ODN comprising SEQ ID NO:1 and an ODN comprising SEQ ID NO:43 compared to the combined immune response of each ODN administered separately.

### Example 4

The following example demonstrates *in vitro* induction of an immune response after administration of multiple ODNs comprising a CpG sequence. Induction of an immune response was measured by production of the cytokines IL-6 and IFN-γ in human PBMC isolated from individual donors as compared to unstimulated PBMC from the same donor.

Human PBMC were isolated, as described in Example 1. ODNs were synthesized on a DNA synthesizer (Applied Biosystems Inc., Foster City, CA), as described in Example 1. In some ODNs, the normal DNA backbone phosphodiesterase linkages were replaced with phosphorothioate linkages, as described in Example 1. To reduce degradation of the ODNs, those that did not have an entire phosphorothioate backbone contained phosphorothioate linkages at the 5' and 3' ends. Cells were incubated for approximately 72 hrs with the various ODNs. IL-6 and IFN-γ levels were determined by ELISA using anti-IL-6 and anti-IFN-γ antibodies, as described in Example 1. The percentage of donors induced by the various multiple ODNs by at least 3-fold for IL-6 and 5-fold for IFN-γ are set forth for IL-6 levels in Table 9: Percent Induction of an Immune Response (IL-6) and for IFN-γ levels in Table 10: Induction of an Immune Response (IFN-γ).

**TABLE 9: Percent Induction of an Immune Response (IL-6) to Multiple ODNs**

| | Percent Induction | Number of Donors |
|---|---|---|
| SEQ ID NO:7 + SEQ ID NO:1 + SEQ ID NO:108 | 100% | 4 |
| SEQ ID NO:7 + SEQ ID NO:20 + SEQ ID NO:98 | 100% | 2 |
| SEQ ID NO:20 + SEQ ID NO:108 + SEQ ID NO:98 | 100% | 2 |
| SEQ ID NO:7 + SEQ ID NO:20 + SEQ ID NO:1 | 80% | 5 |
| SEQ ID NO:20 + SEQ ID NO:1 + SEQ ID NO: 108 | 80% | 5 |
| SEQ ID NO:7 + SEQ ID NO:20 + SEQ ID NO:108 | 60% | 10 |
| SEQ ID NO:7 + SEQ ID NO:108 + SEQ ID NO:98 | 38% | 16 |

**TABLE 10: Percent Induction of an Immune Response (IFN-y) to Multiple ODNs**

| | Percent Induction | Number of Donors |
|---|---|---|
| SEQ ID NO:43 + SEQ ID NO:40 + SEQ ID NO:106 | 100% | 5 |
| SEQ ID NO:32 + SEQ ID NO:40 + SEQ ID NO:106 | 100% | 5 |
| SEQ ID NO:43 + SEQ ID NO:102 + SEQ ID NO:106 | 89% | 19 |
| SEQ ID NO:32 + SEQ ID NO:40 + SEQ ID NO:42 | 80% | 5 |
| SEQ ID NO:32 + SEQ ID NO:102 + SEQ ID NO:106 | 40% | 5 |
| SEQ ID NO:43 + SEQ ID NO:40 + SEQ ID NO:42 | 40% | 5 |

The foregoing data demonstrates the induction of an immune response after administration of multiple ODNs comprising various CpG sequences in human PBMC isolated from individual donors. Specifically, these data demonstrate that multiple ODNs synergistically induce an immune response, as demonstrated by, e.g., Table 9, in which the percent induction was increased in three of the multiple ODNs to 100%, as measured by IL-6 production. This is also show in Table 10, in which the percent induction was increased to 100% for two of the multiple ODNs, as measured by IFN-γ production.

### Example 5

The following example demonstrates *in vitro* induction of an immune response after administration of a single ODN comprising multiple different CpG sequences. Induction of an immune response was measured by production of the cytokines IL-6 and IFN-γ, and cell proliferation in human PBMC isolated from individual donors.

Human PBMC were isolated, as described in Example 1. ODNs were synthesized on a DNA synthesizer (Applied Biosystems Inc., Foster City, CA), as described in Example 1. In some ODNs, the normal DNA backbone phosphodiesterase linkages were replaced with phosphorothioate linkages, as described in Example 1. To reduce degradation of the ODNs, those that did not have an entire phosphorothioate backbone contained phosphorothioate linkages at the 5' and 3' ends. Cells were incubated for approximately 72 hrs with the various ODNs. IL-6 and IFN-γ levels were determined by ELISA using anti-IL-6 and anti-IFN-γ antibodies, as described in Example 1. Cell proliferation was determined by [³H] thymidine incorporation, as described in Example 1. Results are set forth in Table 11: Induction of an Immune Response to a Single ODN Comprising Multiple Different CpG Sequences.

**TABLE 11: Induction of an Immune Response to a Single ODN Comprising Multiple Different CpG Sequences**

| | IL-6 (ELISA) | IFN-γ (ELISA) | Proliferation ([³H] T) |
|---|---|---|---|
| Donor 1 | | | |
| SEQ ID NO:1 | 5 | 3 | 18 |
| SEQ ID NO:43 | 3 | 4 | 3 |
| SEQ ID NO:1 + SEQ ID NO:43 | 23 | 7 | 29 |

| Donor 2 | | | |
|---|---|---|---|
| SEQ ID NO:1 | 5 | 3 | 31 |
| SEQ ID NO:43 | 2 | 4 | 4 |
| SEQ ID NO:1 + SEQ ID NO:43 | 15 | 6 | 57 |

The foregoing data demonstrates the induction of an immune response after administration of a single ODN comprising multiple different CpG sequences in human PBMC isolated from individual donors. Specifically, these data demonstrate that a single ODN comprising multiple different CpG sequences synergistically induces an immune response, such as is demonstrated by, e.g., an increase in IL-6 levels of 65.2% in Donor 1 and 53.3% in Donor 2 after administration of a single ODN comprising SEQ ID NO:1 and SEQ ID NO:43 compared to the combined immune response of a single ODN comprising either SEQ ID NO:1 or SEQ ID NO:43 when administered separately.

## Claims

1. An oligodeoxynucleotide (ODN) comprising multiple CpG sequences, wherein the ODN comprises the CpG sequences set forth in SEQ ID NO: 7 and SEQ ID NO:1 and SEQ ID NO: 108; or SEQ ID NO: 7 and SEQ ID NO: 20 and SEQ ID NO: 98; or SEQ ID NO: 20 and SEQ ID NO: 108 and SEQ ID NO:98; or SEQ ID NO:7 and SEQ ID NO:20 and SEQ ID NO:1; or SEQ ID NO:20 and SEQ ID NO:1 and SEQ ID NO:108; or
multiple oligodeoxynucleotides (ODNs) each comprising a single CpG sequences, wherein the multiple ODNs comprise the CpG sequences set forth in SEQ ID NO: 7 and SEQ ID NO:1 and SEQ ID NO: 108; or SEQ ID NO: 7 and SEQ ID NO: 20 and SEQ ID NO: 98; or SEQ ID NO: 20 and SEQ ID NO: 108 and SEQ ID NO:98; or SEQ ID NO:7 and SEQ ID NO:20 and SEQ ID NO:1; or SEQ ID NO:20 and SEQ ID NO:1 and SEQ ID NO:108, wherein each of the oligodeoxynucleotides is less than 50 nucleotides in length.

2. An ODN delivery complex, comprising
a) an oligodeoxynucleotide comprising a nucleotide sequence set forth as SEQ ID NO: 7, an additional oligodeoxynucleotide comprising a nucleotide sequence set forth as SEQ ID NO: 1, and a further oligodeoxynucleotide comprising a nucleotide sequence set forth as either SEQ ID NO: 108, wherein each of the oligodeoxynucleotides is less than 50 nucleotides in length;
b) an oligodeoxynucleotide comprising a nucleotide sequence set forth as SEQ ID NO: 7, an additional oligodeoxynucleotide comprising a nucleotide sequence set forth as SEQ ID NO: 20, and a further oligodeoxynucleotide comprising a nucleotide sequence set forth as either SEQ ID NO: 98, wherein each of the oligodeoxynucleotides is less than 50 nucleotides in length;
c) an oligodeoxynucleotide comprising a nucleotide sequence set forth as SEQ ID NO: 20, an additional oligodeoxynucleotide comprising a nucleotide sequence set forth as SEQ ID NO: 108, and a further oligodeoxynucleotide comprising a nucleotide sequence set forth as either SEQ ID NO: 98, wherein each of the oligodeoxynucleotides is less than 50 nucleotides in length;
d) an oligodeoxynucleotide comprising a nucleotide sequence set forth as SEQ ID NO: 7, an additional oligodeoxynucleotide comprising a nucleotide sequence set forth as SEQ ID NO: 20, and a further oligodeoxynucleotide comprising a nucleotide sequence set forth as either SEQ ID NO: 1, wherein each of the oligodeoxynucleotides is less than 50 nucleotides in length;
e) an oligodeoxynucleotide comprising a nucleotide sequence set forth as SEQ ID NO: 20, an additional oligodeoxynucleotide comprising a nucleotide sequence set forth as SEQ ID NO: 1, and a further oligodeoxynucleotide comprising a nucleotide sequence set forth as either SEQ ID NO: 108, wherein each of the oligodeoxynucleotides is less than 50 nucleotides in length; or
f) an oligodeoxynucleotide comprising nucleotide sequences set forth in SEQ ID NO: 7 and SEQ ID NO:1 and SEQ ID NO: 108; or SEQ ID NO: 7 and SEQ ID NO: 20 and SEQ ID NO: 98; or SEQ ID NO: 20 and SEQ ID NO: 108 and SEQ ID NO:98; or SEQ ID NO:7 and SEQ ID NO:20 and SEQ ID NO:1; or SEQ ID NO:20 and SEQ ID NO:1 and SEQ ID NO:108.

3. A pharmaceutical composition comprising
a) an oligodeoxynucleotide comprising a nucleotide sequence set forth as SEQ ID NO: 7, an additional oligodeoxynucleotide comprising a nucleotide sequence set forth as SEQ ID NO: 1, and a further oligodeoxynucleotide comprising a nucleotide sequence set forth as either SEQ ID NO: 108, wherein each of the oligodeoxynucleotides is less than 50 nucleotides in length;
b) an oligodeoxynucleotide comprising a nucleotide sequence set forth as SEQ ID NO: 7, an additional oligodeoxynucleotide comprising a nucleotide sequence set forth as SEQ ID NO: 20, and a further oligodeoxynucleotide comprising a nucleotide sequence set forth as either SEQ ID NO: 98, wherein each of the oligodeoxynucleotides is less than 50 nucleotides in length;
c) an oligodeoxynucleotide comprising a nucleotide sequence set forth as SEQ ID NO: 20, an additional oligodeoxynucleotide comprising a nucleotide sequence set forth as SEQ ID NO: 108, and a further oligodeoxynucleotide comprising a nucleotide sequence set forth as either SEQ ID NO: 98, wherein each of the oligodeoxynucleotides is less than 50 nucleotides in length;
d) an oligodeoxynucleotide comprising a nucleotide sequence set forth as SEQ ID NO: 7, an additional oligodeoxynucleotide comprising a nucleotide sequence set forth as SEQ ID NO: 20, and a further oligodeoxynucleotide comprising a nucleotide sequence set forth as either SEQ ID NO: 1, wherein each of the oligodeoxynucleotides is less than 50 nucleotides in length;
e) an oligodeoxynucleotide comprising a nucleotide sequence set forth as SEQ ID NO: 20, an additional oligodeoxynucleotide comprising a nucleotide sequence set forth as SEQ ID NO: 1, and a further oligodeoxynucleotide comprising a nucleotide sequence set forth as either SEQ ID NO: 108, wherein each of the oligodeoxynucleotides is less than 50 nucleotides in length; or
f) an oligodeoxynucleotide comprising nucleotide sequences set forth in SEQ ID NO: 7 and SEQ ID NO:1 and SEQ ID NO: 108; or SEQ ID NO: 7 and SEQ ID NO: 20 and SEQ ID NO: 98; or SEQ ID NO: 20 and SEQ ID NO: 108 and SEQ ID NO:98; or SEQ ID NO:7 and SEQ ID NO:20 and SEQ ID NO:1; or SEQ ID NO:20 and SEQ ID NO:1 and SEQ ID NO:108; and/or
g) and a pharmaceutically acceptable carrier.

4. The ODN or ODNs of Claim 1, ODN complex of Claim 2 of or pharmaceutical composition of Claim 3, wherein the ODN has, or the ODNs have, a phosphate backbone modification.

5. The ODN or ODNs, ODN complex or pharmaceutical composition of Claim 4, wherein the phosphate backbone modification is a phosphorothioate backbone modification.

6. The ODN or ODNs of any one of Claims 1 and 4 to 5, ODN complex of any one of Claim 2 and 4 to 5 and pharmaceutical composition of any one of Claims 3 and 4 to 5, wherein the ODN is, or ODNs are, modified to prevent degradation.

7. The ODN or ODNs of any one of Claims 1 and 4 to 6, wherein comprises 100 nucleotides or less.

8. The ODN of Claim 7, wherein the ODN comprises 50 nucleotides or less.

9. The ODN or ODNs of any one of Claims 1 and 4 to 8, ODN complex of any one of Claims 2 and 4 to 6 and pharmaceutical composition of any one of Claims 3 and 4 to 6 for use in a therapeutic method of inducing an immune response.

10. The use of the ODN or ODNs of any one of Claims 1 and 4 to 8, ODN complex of any one of Claim 2 and 4 to 6 and pharmaceutical composition of any one of Claims 3 and 4 to 6 for the manufacture of a medicament for the treatment, amelioration or prevention of an autoimmune disorder or an immune system deficiency.

11. The use of the ODN or ODNs of any one of Claims 1 and 4 to 8, ODN complex of any one of Claim 2 and 4 to 6 and pharmaceutical composition of any one of Claims 3 and 4 to 6 for the manufacture of a medicament for the treatment, amelioration or prevention of a disease by inducing an immune response by the release of cytokines.

12. The use of any one of Claims 10 or 11 wherein the medicament is used to treat, prevent or ameliorate an allergic reaction, and, optionally, is suitable for administration in combination with an anti-allergenic agent.

13. The use of Claim 12, wherein the allergic reaction is eczema, allergic rhinitis or coryza, hay fever, bronchial asthma, uticaria (hives), food allergies.

14. The use of Claim 12, wherein the allergic reaction is brought about by an allergen selected from the group consisting of pollens, insect venoms, animal dander, dust, fungal spores and penicillins.

15. The use of Claim 12, wherein the medicament is used to treat allergic asthma.

16. The use of any one of Claim 10 or 11, wherein the medicament is for treating cancer, optionally, in combination with an anti-cancer agent.

17. The use of Claim 16, wherein the cancer is selected from the group consisting of cancers of the brain, lung, ovary, breast, prostate and colon as well as carcinomas and sarcomas .

18. The use of Claim 16, wherein the cancer is a solid tumour cancer.

19. The use of any one of Claims 10 or 11, wherein the immune system deficiency is lupus erythematosus.

20. The use of any one of Claims 10 or 11, wherein the autoimmune disease is rheumatoid arthritis or multiple sclerosis.

21. The use of any one of Claims 10 or 11, wherein the medicament is for treating infections selected from the group consisting of francisella, schistosomiasis, tuberculosis, AIDS, malaria and leishmania.

22. The use of Claim 21, wherein the medicament is suitable to be used in combination with any anti-infectious agent.

23. The use of any one of Claim 10 or 11, wherein the medicament is for treating, preventing or ameliorating symptoms resulting from exposure to a bio-warfare agent.

24. The use of Claim 23, wherein the bio-warfare agent is a biological agent selected from the group consisting of Ebola, Anthrax and Listeria.

## Patentansprüche

1. Oligodesoxynukleotid (ODN), das mehrere CpG-Sequenzen umfasst, wobei das ODN die in SEQ ID Nr. 7 und SEQ ID Nr. 1 und SEQ ID Nr. 108; oder SEQ ID Nr. 7 und SEQ ID Nr. 20 und SEQ ID Nr. 98; oder SEQ ID Nr. 20 und SEQ ID Nr. 108 und SEQ ID Nr. 98; oder SEQ ID Nr. 7 und SEQ ID Nr. 20 und SEQ ID Nr. 1; oder SEQ ID Nr. 20 und SEQ ID Nr. 1 und SEQ ID Nr. 108 dargelegten CpG-Sequenzen umfasst; oder
mehrere Oligodesoxynukleotide (ODNs), die jeweils eine einzige CpG-Sequenz umfassen, wobei die mehreren ODNs die in SEQ ID Nr. 7 und SEQ ID Nr. 1 und SEQ ID Nr. 108; oder SEQ ID Nr. 7 und SEQ ID Nr. 20 und SEQ ID Nr. 98; oder SEQ ID Nr. 20 und SEQ ID Nr. 108 und SEQ ID Nr. 98; oder SEQ ID Nr. 7 und SEQ ID Nr. 20 und SEQ ID Nr. 1; oder SEQ ID Nr. 20 und SEQ ID Nr. 1 und SEQ ID Nr. 108 dargelegten CpG-Sequenzen umfassen, wobei jedes der Oligodesoxynukleotide weniger als 50 Nukleotide lang ist.

2. ODN-Abgabekomplex, der Folgendes umfasst:
a) ein Oligodesoxynukleotid, das eine als SEQ ID Nr. 7 dargelegte Nukleotidsequenz umfasst, ein zusätzliches Oligodesoxynukleotid, das eine als SEQ ID Nr. 1 dargelegte Nukleotidsequenz umfasst, und ein weiteres Oligodesoxynukleotid, das eine als entweder SEQ ID Nr. 108 dargelegte Nukleotidsequenz umfasst, wobei jedes der Oligodesoxynukleotide weniger als 50 Nukleotide lang ist;
b) ein Oligodesoxynukleotid, das eine als SEQ ID Nr. 7 dargelegte Nukleotidsequenz umfasst, ein zusätzliches Oligodesoxynukleotid, das eine als SEQ ID Nr. 20 dargelegte Nukleotidsequenz umfasst, und ein weiteres Oligodesoxynukleotid, das eine als entweder SEQ ID Nr. 98 dargelegte Nukleotidsequenz umfasst, wobei jedes der Oligodesoxynukleotide weniger als 50 Nukleotide lang ist;
c) ein Oligodesoxynukleotid, das eine als SEQ ID Nr. 20 dargelegte Nukleotidsequenz umfasst, ein zusätzliches Oligodesoxynukleotid, das eine als SEQ ID Nr. 108 dargelegte Nukleotidsequenz umfasst, und ein weiteres Oligodesoxynukleotid, das eine als entweder SEQ ID Nr. 98 dargelegte Nukleotidsequenz umfasst, wobei jedes der Oligodesoxynukleotide weniger als 50 Nukleotide lang ist;
d) ein Oligodesoxynukleotid, das eine als SEQ ID Nr. 7 dargelegte Nukleotidsequenz umfasst, ein zusätzliches Oligodesoxynukleotid, das eine als SEQ ID Nr. 20 dargelegte Nukleotidsequenz umfasst, und ein weiteres Oligodesoxynukleotid, das eine als entweder SEQ ID Nr. 1 dargelegte Nukleotidsequenz umfasst, wobei jedes der Oligodesoxynukleotide weniger als 50 Nukleotide lang ist;
e) ein Oligodesoxynukleotid, das eine als SEQ ID Nr. 20 dargelegte Nukleotidsequenz umfasst, ein zusätzliches Oligodesoxynukleotid, das eine als SEQ ID Nr. 1 dargelegte Nukleotidsequenz umfasst, und ein weiteres Oligodesoxynukleotid, das eine als entweder SEQ ID Nr. 108 dargelegte Nukleotidsequenz umfasst, wobei jedes der Oligodesoxynukleotide weniger als 50 Nukleotide lang ist; oder
f) ein Oligodesoxynukleotid, das in SEQ ID Nr. 7 und SEQ ID Nr. 1 und SEQ ID Nr. 108; oder SEQ ID Nr. 7 und SEQ ID Nr. 20 und SEQ ID Nr. 98; oder SEQ ID Nr. 20 und SEQ ID Nr. 108 und SEQ ID Nr. 98; oder SEQ ID Nr. 7 und SEQ ID Nr. 20 und SEQ ID Nr. 1; oder SEQ ID Nr. 20 und SEQ ID Nr. 1 und SEQ ID Nr. 108 dargelegte Nukleotidsequenzen umfasst.

3. Pharmazeutische Zusammensetzung, die Folgendes umfasst:
a) ein Oligodesoxynukleotid, das eine als SEQ ID Nr. 7 dargelegte Nukleotidsequenz umfasst, ein zusätzliches Oligodesoxynukleotid, das eine als SEQ ID Nr. 1 dargelegte Nukleotidsequenz umfasst, und ein weiteres Oligodesoxynukleotid, das eine als entweder SEQ ID Nr. 108 dargelegte Nukleotidsequenz umfasst, wobei jedes der Oligodesoxynukleotide weniger als 50 Nukleotide lang ist;
b) ein Oligodesoxynukleotid, das eine als SEQ ID Nr. 7 dargelegte Nukleotidsequenz umfasst, ein zusätzliches Oligodesoxynukleotid, das eine als SEQ ID Nr. 20 dargelegte Nukleotidsequenz umfasst, und ein weiteres Oligodesoxynukleotid, das eine als entweder SEQ ID Nr. 98 dargelegte Nukleotidsequenz umfasst, wobei jedes der Oligodesoxynukleotide weniger als 50 Nukleotide lang ist;
c) ein Oligodesoxynukleotid, das eine als SEQ ID Nr. 20 dargelegte Nukleotidsequenz umfasst, ein zusätzliches Oligodesoxynukleotid, das eine als SEQ ID Nr. 108 dargelegte Nukleotidsequenz umfasst, und ein weiteres Oligodesoxynukleotid, das eine als entweder SEQ ID Nr. 98 dargelegte Nukleotidsequenz umfasst, wobei jedes der Oligodesoxynukleotide weniger als 50 Nukleotide lang ist;
d) ein Oligodesoxynukleotid, das eine als SEQ ID Nr. 7 dargelegte Nukleotidsequenz umfasst, ein zusätzliches Oligodesoxynukleotid, das eine als SEQ ID Nr. 20 dargelegte Nukleotidsequenz umfasst, und ein weiteres Oligodesoxynukleotid, das eine als entweder SEQ ID Nr. 1 dargelegte Nukleotidsequenz umfasst, wobei jedes der Oligodesoxynukleotide weniger als 50 Nukleotide lang ist;
e) ein Oligodesoxynukleotid, das eine als SEQ ID Nr. 20 dargelegte Nukleotidsequenz umfasst, ein zusätzliches Oligodesoxynukleotid, das eine als SEQ ID Nr. 1 dargelegte Nukleotidsequenz umfasst, und ein weiteres Oligodesoxynukleotid, das eine als entweder SEQ ID Nr. 108 dargelegte Nukleotidsequenz umfasst, wobei jedes der Oligodesoxynukleotide weniger als 50 Nukleotide lang ist; oder
f) ein Oligodesoxynukleotid, das in SEQ ID Nr. 7 und SEQ ID Nr. 1 und SEQ ID Nr. 108; oder SEQ ID Nr. 7 und SEQ ID Nr. 20 und SEQ ID Nr. 98; oder SEQ ID Nr. 20 und SEQ ID Nr. 108 und SEQ ID Nr. 98; oder SEQ ID Nr. 7 und SEQ ID Nr. 20 und SEQ ID Nr. 1; oder SEQ ID Nr. 20 und SEQ ID Nr. 1 und SEQ ID Nr. 108 dargelegte Nukleotidsequenzen umfasst; und/oder
g) einen pharmazeutisch unbedenklichen Trägerstoff.

4. ODN oder ODNs nach Anspruch 1, ODN-Komplex nach Anspruch 2 oder pharmazeutische Zusammensetzung nach Anspruch 3, wobei das ODN oder die ODN eine Phosphat-Rückgratmodifikation aufweist bzw. aufweisen.

5. ODN oder ODNs, ODN-Komplex oder pharmazeutische Zusammensetzung nach Anspruch 4, wobei die Phosphat-Rückgratmodifikation eine Phosphorthioat-Rückgratmodifikation ist.

6. ODN oder ODNs nach einem der Ansprüche 1 und 4 bis 5, ODN-Komplex nach einem der Ansprüche 2 und 4 bis 5 und pharmazeutische Zusammensetzung nach einem der Ansprüche 3 und 4 bis 5, wobei das ODN oder die ODN modifiziert ist bzw. sind, um einen Abbau zu verhindern.

7. ODN oder ODNs nach einem der Ansprüche 1 und 4 bis 6, das bzw. die 100 Nukleotide oder weniger umfasst bzw. umfassen.

8. ODN nach Anspruch 7, wobei das ODN 50 Nukleotide oder weniger umfasst.

9. ODN oder ODNs nach einem der Ansprüche 1 und 4 bis 8, ODN-Komplex nach einem der Ansprüche 2 und 4 bis 6 und pharmazeutische Zusammensetzung nach einem der Ansprüche 3 und 4 bis 6 zur Verwendung in einem Therapieverfahren zum Induzieren einer Immunantwort.

10. Verwendung des ODN oder der ODNs nach einem der Ansprüche 1 und 4 bis 8, des ODN-Komplexes nach einem der Ansprüche 2 und 4 bis 6 und der pharmazeutischen Zusammensetzung nach einem der Ansprüche 3 und 4 bis 6 zur Herstellung eines Arzneimittels zur Behandlung, Linderung oder Prävention einer Autoimmunerkrankung oder einer Immunsystemschwäche.

11. Verwendung des ODN oder der ODNs nach einem der Ansprüche 1 und 4 bis 8, des ODN-Komplexes nach einem der Ansprüche 2 und 4 bis 6 und der pharmazeutischen Zusammensetzung nach einem der Ansprüche 3 und 4 bis 6 zur Herstellung eines Arzneimittels zur Behandlung, Linderung oder Prävention einer Krankheit durch Induzieren einer Immunantwort durch das Freisetzen von Zytokinen.

12. Verwendung nach einem der Ansprüche 10 oder 11, wobei das Arzneimittel dazu verwendet wird, eine allergische Reaktion zu behandeln, zu verhindern oder zu lindern, und gegebenenfalls zur Verabreichung in Kombination mit einem Antiallergikum geeignet ist.

13. Verwendung nach Anspruch 12, wobei es sich bei der allergischen Reaktion um ein Ekzem, Rhinitis allergica oder Rhinitis acuta, Heuschnupfen, Bronchialasthma, Urtikaria (Nesselsucht) oder Lebensmittelallergien handelt.

14. Verwendung nach Anspruch 12, wobei die allergische Reaktion von einem Allergen verursacht wird, das aus der Gruppe bestehend aus Pollen, Insektengiften, Tierschuppen, Staub, Pilzsporen und Penicillinen ausgewählt ist.

15. Verwendung nach Anspruch 12, wobei das Arzneimittel dazu verwendet wird, allergisches Asthma zu behandeln.

16. Verwendung nach einem der Ansprüche 10 oder 11, wobei das Arzneimittel zum Behandeln einer Krebserkrankung ist, gegebenenfalls in Kombination mit einem antineoplastischen Mittel.

17. Verwendung nach Anspruch 16, wobei die Krebserkrankung aus der Gruppe bestehend aus Krebserkrankungen der Brust, der Lunge, der Eierstöcke, der Prostata und des Dickdarms sowie Karzinomen und Sarkomen ausgewählt ist.

18. Verwendung nach Anspruch 16, wobei es sich bei der Krebserkrankung um einen soliden Tumor handelt.

19. Verwendung nach einem der Ansprüche 10 oder 11, wobei es sich bei der Immunsystemschwäche um Lupus erythematodes handelt.

20. Verwendung nach einem der Ansprüche 10 oder 11, wobei es sich bei der Autoimmunerkrankung um rheumatoide Arthritis oder multiple Sklerose handelt.

21. Verwendung nach einem der Ansprüche 10 oder 11, wobei das Arzneimittel zum Behandeln von Infektionen ist, die aus der Gruppe bestehend aus Francisella, Schistosomiasis, Tuberkulose, AIDS, Malaria und Leishmania ausgewählt ist.

22. Verwendung nach Anspruch 21, wobei das Arzneimittel dazu geeignet ist, in Kombination mit einem beliebigen Antiinfektiosum verwendet zu werden.

23. Verwendung nach einem der Ansprüche 10 oder 11, wobei das Arzneimittel zum Behandeln, Verhindern oder Lindern von Symptomen ist, die aus der Exposition mit einem biologischen Kampfstoff resultiert.

24. Verwendung nach Anspruch 23, wobei der biologische Kampfstoff ein biologischer Stoff ist, der aus der Gruppe bestehend aus Ebola, Anthrax und Listeria ausgewählt ist.

## Revendications

1. Oligodésoxynucléotide (ODN) comprenant plusieurs séquences CpG, l'ODN comprenant les séquences CpG conformes à ID SÉQ N° 7 et ID SÉQ N° 1 et ID SÉQ N°108; ou ID SÉQ N°7 et ID SÉQ N° 20 et ID SÉQ N°98 ; ou ID SÉQ N° 20 et ID SÉQ N°108 et ID SÉQ N° 98 ; ou ID SÉQ N°7 et ID SÉQ N° 20 et ID SÉQ N°1 ; ou ID SÉQ N° 20 et ID SÉQ N°1 et ID SÉQ N° 108 ; ou
plusieurs oligodésoxynucléotides (ODNs) comprenant chacun une seule séquences CpG, les plusieurs ODN comprenant les séquences CpG conformes à ID SÉQ N° 7 et ID SÉQ N° 1 et ID SÉQ NO 108 ; ou ID SÉQ NO 7 et ID SÉQ NO 20 et ID SÉQ NO 98 ou ID SÉQ N° 20 et ID SÉQ N° 108 et ID SÉQ NO 98 ; ou ID SÉQ NO 7 et ID SÉQ NO 20 et ID SÉQ N° 1 ; ou ID SÉQ N° 20 et ID SÉQ N° 1 et ID SÉQ N° 108 ; chacun des oligodésoxynucléotides étant inférieur à 50 nucléotides en longueur.

2. Complexe de distribution d'ODN, comprenant
a) un oligodésoxynucléotide comprenant une séquence de nucléotides conforme à ID SÉQ N° 7, un oligodésoxynucléotide supplémentaire comprenant une séquence de nucléotides conforme à ID SÉQ N° 1, et un autre oligodésoxynucléotide comprenant une séquence de nucléotides conforme à ID SÉQ N° 108, chacun des oligodésoxynucléotides étant inférieur à 50 nucléotides en longueur ;
b) un oligodésoxynucléotide comprenant une séquence de nucléotides conforme à ID SÉQ N° 7, un oligodésoxynucléotide supplémentaire comprenant une séquence de nucléotides conforme à ID SÉQ N° 20, et un autre oligodésoxynucléotide comprenant une séquence de nucléotides conforme à ID SÉQ N° 98, chacun des oligodésoxynucléotides étant inférieur à 50 nucléotides en longueur ;
c) un oligodésoxynucléotide comprenant une séquence de nucléotides conforme à ID SÉQ N° 20, un oligodésoxynucléotide supplémentaire comprenant une séquence de nucléotides conforme à ID SÉQ N° 108, et un autre oligodésoxynucléotide comprenant une séquence de nucléotides conforme à ID SÉQ N° 98, chacun des oligodésoxynucléotides étant inférieur à 50 nucléotides en longueur ;
d) un oligodésoxynucléotide comprenant une séquence de nucléotides conforme à ID SÉQ N° 7, un oligodésoxynucléotide supplémentaire comprenant une séquence de nucléotides conforme à ID SÉQ N° 20, et un autre oligodésoxynucléotide comprenant une séquence de nucléotides conforme à ID SÉQ N° 1, chacun des oligodésoxynucléotides étant inférieur à 50 nucléotides en longueur ;
e) un oligodésoxynucléotide comprenant une séquence de nucléotides conforme à ID SÉQ N° 20, un oligodésoxynucléotide supplémentaire comprenant une séquence de nucléotides conforme à ID SÉQ N° 1, et un autre oligodésoxynucléotide comprenant une séquence de nucléotides conforme à ID SÉQ N° 108, chacun des oligodésoxynucléotides étant inférieur à 50 nucléotides en longueur ; ou
f) oligodésoxynucléotide comprenant les séquences de nucléotides conformes à ID SÉQ N° 7 et ID SÉQ N° 1 et ID SÉQ N° 108 ; ou ID SÉQ N° 7 et ID SÉQ N° 20 et ID SÉQ N°98 ; ou ID SÉQ N°20 et ID SÉQ N° 108 et ID SÉQ N° 98; ou ID SÉQ N°7 et ID SÉQ N° 20 et ID SÉQ NO 1 ; ou ID SÉQ N° 20 et ID SÉQ N° 1 et ID SÉQ N° 108.

3. Composition pharmaceutique comprenant :
a) un oligodésoxynucléotide comprenant une séquence de nucléotides conforme à ID SÉQ N° 7, un oligodésoxynucléotide supplémentaire comprenant une séquence de nucléotides conforme à ID SÉQ N° 1, et un autre oligodésoxynucléotide comprenant une séquence de nucléotides conforme à ID SÉQ N° 108, chacun des oligodésoxynucléotides étant inférieur à 50 nucléotides en longueur ;
b) un ligodésoxynucléotide comprenant une séquence de nucléotides conforme à ID SÉQ N° 7, un oligodésoxynucléotide supplémentaire comprenant une séquence de nucléotides conforme à ID SÉQ N° 20, et un autre oligodésoxynucléotide comprenant une séquence de nucléotides conforme à ID SÉQ N° 98, chacun des oligodésoxynucléotides étant inférieur à 50 nucléotides en longueur ;
c) un oligodésoxynucléotide comprenant une séquence de nucléotides conforme à ID SÉQ N° 20, un oligodésoxynucléotide supplémentaire comprenant une séquence de nucléotides conforme à ID SÉQ N° 108, et un autre oligodésoxynucléotide comprenant une séquence de nucléotides conforme à ID SÉQ N° 98, chacun des oligodésoxynucléotides étant inférieur à 50 nucléotides en longueur ;
d) un oligodésoxynucléotide comprenant une séquence de nucléotides conforme à ID SÉQ N° 7, un oligodésoxynucléotide supplémentaire comprenant une séquence de nucléotides conforme à ID SÉQ N° 20, et un autre oligodésoxynucléotide comprenant une séquence de nucléotides conforme à ID SÉQ N° 1, chacun des oligodésoxynucléotides étant inférieur à 50 nucléotides en longueur ;
e) un oligodésoxynucléotide comprenant une séquence de nucléotides conforme à ID SÉQ N° 20, un oligodésoxynucléotide supplémentaire comprenant une séquence de nucléotides conforme à ID SÉQ N° 1, et un autre oligodésoxynucléotide comprenant une séquence de nucléotides conforme à ID SÉQ N° 108, chacun des oligodésoxynucléotides étant inférieur à 50 nucléotides en longueur ; ou
f) oligodésoxynucléotide comprenant les séquences de nucléotides conformes à ID SÉQ N° 7 et ID SÉQ N° 1 et ID SÉQ N° 108 ; ou ID SÉQ N° 7 et ID SÉQ N° 20 et ID SÉQ N° 98 ; ou ID SÉQ N° 20 et ID SÉQ N° 108 et ID SÉQ N° 98 ; ou ID SÉQ N° 7 et ID SÉQ N° 20 et ID SÉQ N° 1 ; ou ID SÉQ N° 20 et ID SÉQ N° 1 et ID SÉQ N° 108 ; et/ou
g) support pharmaceutiquement acceptable.

4. Oligodésoxynucléotide ou oligodésoxynucléotides (ODN) selon la revendication 1, complexe d'ODN selon la revendication 2 ou composition pharmaceutique selon la revendication 3, où le ou les ODN présentent une modification du squelette phosphate.

5. Oligodésoxynucléotide ou oligodésoxynucléotides (ODN), complexe d'ODN ou composition pharmaceutique selon la revendication 4, où la modification du squelette phosphate est une modification du squelette phosphorothioate.

6. Oligodésoxynucléotide ou oligodésoxynucléotides (ODN) selon l'une quelconque des revendications 1 et 4 à 5, complexe d'ODN selon l'une quelconque des revendications 2 et 4 à 5, et composition pharmaceutique selon l'une quelconque des revendications 3 et 4 à 5, où le ou les ODN sont modifiés pour prévenir une dégradation.

7. Oligodésoxynucléotide ou oligodésoxynucléotides (ODN) selon l'une quelconque des revendications 1 et 4 à 6, comprenant 100 nucléotides ou moins.

8. ODN selon la revendication 7, l'ODN comprenant 50 nucléotides ou moins.

9. Oligodésoxynucléotide ou oligodésoxynucléotides (ODN) selon l'une quelconque des revendications 1 et 4 à 8, complexe d'ODN selon l'une quelconque des revendications 2 et 4 à 6, et composition pharmaceutique selon l'une quelconque des revendications 3 et 4 à 6, destinés à être utilisés dans un procédé thérapeutique consistant à induire une réponse immunitaire.

10. Utilisation de l'oligodésoxynucléotide ou des oligodésoxynucléotides (ODN) selon l'une quelconque des revendications 1 et 4 à 8, du complexe d'ODN selon l'une quelconque des revendications 2 et 4 à 6, et de la composition pharmaceutique selon l'une quelconque des revendications 3 et 4 à 6 pour la fabrication d'un médicament pour le traitement, l'amélioration ou la prévention d'un trouble auto-immune ou d'un déficit du système immunitaire.

11. Utilisation de l'oligodésoxynucléotide ou des oligodésoxynucléotides (ODN) selon l'une quelconque des revendications 1 et 4 à 8, du complexe d'ODN selon l'une quelconque des revendications 2 et 4 à 6, et de la composition pharmaceutique selon l'une quelconque des revendications 3 et 4 à 6 pour la fabrication d'un médicament pour le traitement, l'amélioration ou la prévention d'une maladie en induisant une réponse immunitaire en libérant des cytokines.

12. Utilisation selon l'une quelconque des revendications 10 et 11, dans laquelle le médicament est utilisé pour traiter, prévenir ou améliorer une réaction allergique et, éventuellement, est adapté pour être administré en combinaison avec un agent antiallergique.

13. Utilisation selon la revendication 12, dans laquelle la réaction allergique est un eczéma, une rhinite allergique ou un coryza, un rhume des foins, de l'asthme bronchique, de l'urticaire ou des allergies alimentaires.

14. Utilisation selon la revendication 12, dans laquelle la réaction allergique est provoquée par un allergène choisi dans le groupe constitué de pollens, de venins d'insecte, de squames animales, de poussière, de spores fongiques et de pénicillines.

15. Utilisation selon la revendication 12, dans laquelle le médicament est utilisé pour traiter l'asthme allergique.

16. Utilisation selon l'une quelconque des revendications 10 et 11, dans laquelle le médicament est destiné à traiter un cancer, éventuellement en combinaison avec un agent anticancéreux.

17. Utilisation selon la revendication 16, dans laquelle le cancer est choisi dans le groupe constitué de cancers du cerveau, du poumon, de l'ovaire, du sein, de la prostate et du colon, ainsi que des carcinomes et des sarcomes.

18. Utilisation selon la revendication 16, dans laquelle le cancer est un cancer de type tumeur solide.

19. Utilisation selon l'une quelconque des revendications 10 et 11, dans laquelle le déficit du système immunitaire est le lupus érythémateux.

20. Utilisation selon l'une quelconque des revendications 10 et 11, dans laquelle la maladie auto-immune est une polyarthrite rhumatoïde ou une sclérose en plaques.

21. Utilisation selon l'une quelconque des revendications 10 et 11, dans laquelle le médicament est destiné à traiter des infections choisies dans le groupe constitué de la tularémie, la schistosomiase, la tuberculose, le SIDA, la malaria et la leishmaniose.

22. Utilisation selon la revendication 21, dans laquelle le médicament convient pour être utilisé en combinaison avec un agent anti-infectieux.

23. Utilisation selon l'une quelconque des revendications 10 et 11, dans laquelle le médicament est destiné à traiter, prévenir ou améliorer les symptômes résultant d'une exposition à un agent de guerre biologique.

24. Utilisation selon la revendication 23, dans laquelle l'agent de guerre biologique est un agent biologique choisi dans le groupe constitué de l'Ébola, de l'anthrax et de la Listeria.
